(19) Europäisches Patentamt
European Patent Office
Office européen des brevets

(11) Publication number: **0 078 671**
**A2**

(12) **EUROPEAN PATENT APPLICATION**

(21) Application number: 82305743.5

(22) Date of filing: 28.10.82

(51) Int. Cl.³: C 07 D 501/22, C 07 D 501/12, A 61 K 31/545

(30) Priority: 30.10.81 US 316615

(43) Date of publication of application: 11.05.83 Bulletin 83/19

(84) Designated Contracting States: BE CH DE FR GB IT LI LU NL SE

(71) Applicant: ELI LILLY AND COMPANY, 307, East McCarty Street, Indianapolis Indiana 46285 (US)

(72) Inventor: Yang, Kuo Shang, 340 Green Hill Court, Greenwood Indiana 46142 (US)

(74) Representative: Hudson, Christopher Mark et al, Erl Wood Manor, Windlesham Surrey GU20, 6PH (GB)

(54) **Novel cephalosporin salt and its use in a purification process.**

(57) Syn-7-‹[(2-amino-4-thiazolyl) (methoxyimino)-acetyl]-amino›-3-methyl-3-cephem-4-carboxylic acid hydrochloride is prepared by mixing the corresponding free base wherein the oxime is in the syn configuration with hydrochloric acid in a solvent, and separating crystals of the salt. The process is useful in purification of the free base.

EP 0 078 671 A2

# NOVEL CEPHALOSPORIN SALT AND ITS USE IN A PURIFICATION PROCESS

This invention relates to cephalosporin derivatives and to methods of purifying cephalosporin antibiotics. More particularly, the invention relates to a novel crystalline salt of the cephalosporin antibiotic of the formula

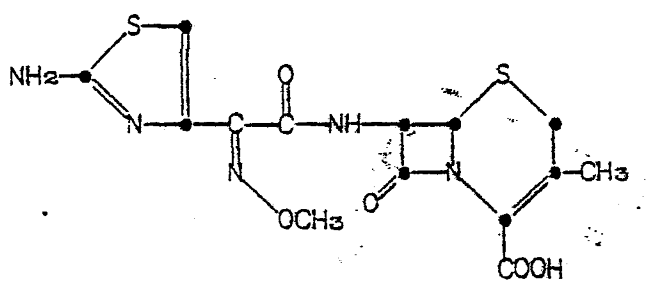

(I)

wherein the oxime is in the _syn_ configuration, and to a method of purifying the cephalosporin of formula (I) utilizing the novel crystalline salt.

The cephalosporin antibiotic of formula (I) is described by Heymes et al. in U.S. Patent 4,202,893, issued May 13, 1980, and by Ochiai et al. in U.S. Patent 4,205,180, issued May 27, 1980 and in U.S. Patent 4,278,671, issued July 14, 1981.

Often the pharmaceutical form of a cephalosporin antibiotic is a salt form. Crystalline salts are especially advantageous because such salts are more stable in addition to being compatible with and soluble in physiological fluids. A further advantage of a crystalline salt form of a cephalosporin is that crystalline substances aid greatly in purifying materials to obtain compounds in substantially pure form. Puri-

fication is essential to obtain products of pharmaceutically-acceptable quality. With cephalosporin antibiotics such as the compound of formula (I), purification procedures are both time-consuming and expensive.

Certain crystalline cephalosporin salts, including crystalline hydrochlorides, have been described [see, for example, West German Patent 2,949,485 (Derwent Abstract 45382C/26), Belgian Patent 885,488 (Derwent Abstract 27245D/16) and U.K. Patent 1,589,841, published May 20, 1981]. The ability to form useful crystalline salts, however, is not predictable.

My invention specifically provides a crystalline hydrochloride salt of the formula

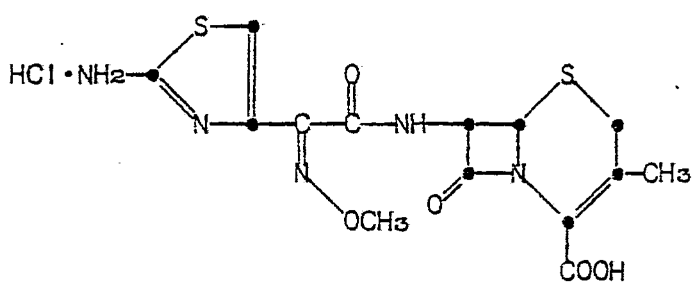
(II)

wherein the oxime is in the syn configuration.

My invention also provides a process for preparing a crystalline hydrochloride salt of the formula

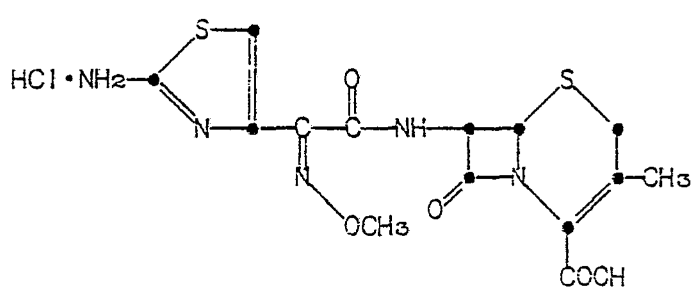
(II)

wherein the oxime is in the syn configuration, which comprises the steps of

(a) mixing a compound of the formula

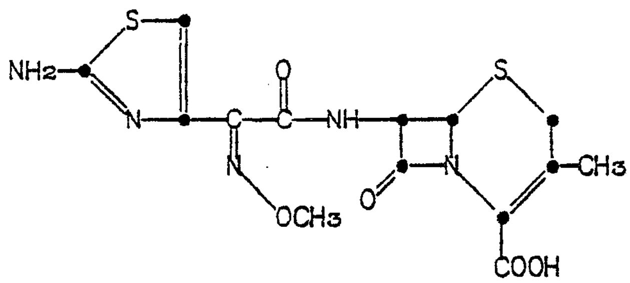

(I)

wherein the oxime is in the syn configuration, with hydrochloric acid in a solvent selected from water, polar organic solvents and mixtures thereof; and

(b) separating crystals of the hydrochloride salt of formula (I) from the mixture.

A further significant aspect of my invention is a process for purifying an impure lot of compound of the formula

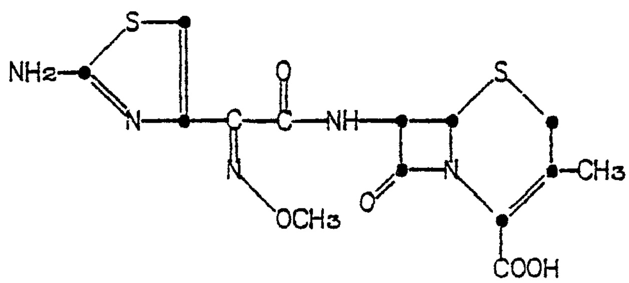

(I)

wherein the oxime is in the syn configuration which comprises:

(a) mixing the impure lot with hydrochloric acid in a solvent selected from water, polar organic solvents, and mixtures thereof;

(b) separating from the mixture crystals of the hydrochloride salt of the formula

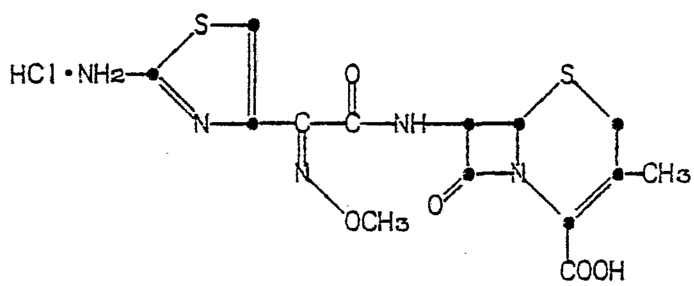

(II)

wherein the oxime is in the syn configuration;

(c) dissolving the crystalline hydrochloride salt of formula (II) in an aqueous bicarbonate solution with the pH maintained between about 6 and about 7;

(d) adding hydrochloric acid to the solution to precipitate the compound of formula (I); and

(e) separating the compound of formula (I) from the mixture.

My discovery of the compound of formula (I) in crystalline form, therefore, provides methods of obtaining both the hydrochloride of formula (II) and the free base of formula (I) in substantially pure form.

The hydrochloride salt of this invention is formally named syn-7-[[(2-amino-4-thiazolyl)(methoxyimino)acetyl]amino]-3-methyl-3-cephem-4-carboxylic acid hydrochloride. For convenience herein this compound is referred to as the hydrochloride salt.

The crystalline hydrochloride salt of this invention, when crystallized from formamide-water, has the following X-ray powder diffraction pattern ($Cu^{++}$ radiation, 1.5418λ, nickel filter, d = interplanar spacing in angstroms):

| d | Relative Intensity |
|---|---|
| 13.56 | 25 |
| 8.61 | 25 |
| 7.78 | 75 |
| 6.88 | 45 |
| 5.80 | 15 |
| 5.13 | 7 |
| 4.42 | 100 |
| 4.29 | 35 |
| 4.10 | 55 |
| 3.98 | 35 |
| 3.87 | 40 |
| 3.64 | 35 |
| 3.47 | 60 |
| 3.16 | 62 |
| 3.07 | 18 |
| 3.00 | 15 |
| 2.91 | 15 |
| 2.80 | 18 |
| 2.74 | 22 |
| 2.60 | 12 |
| 2.50 | 20 |
| 2.45 | 20 |
| 2.39 | 15 |
| 2.23 | 15 |
| 2.15 | 15 |
| 2.12 | 15 |
| 2.00 | 20 |

The crystalline hydrochloride salt provided by this invention is a suitable pharmaceutical form which is stable at ordinary conditions of temperature and humidity. The salt can be stored in bulk form for later use, for example, in preparing unit-dosage forms in ampoules.

According to the process of this invention for preparing the crystalline hydrochloride salt, the free base of formula (I) is dissolved or slurried in water or a polar organic solvent, warming if necessary. The pH of this solution is adjusted to between about pH 1.5 and about 3.5 by the addition of hydrochloric acid. When the hydrochloride salt crystallizes, it is separated by standard methods.

The concentration of the free base of formula (I) in the solvent prior to the addition of the hydrochloric acid is preferably between about 15% and about 20% by weight; however, concentrations between about 10% and about 25% can also be used. When an organic solvent such as formamide is used, it is preferable to dilute the solution of the free base with water, preferably so that compound of formula (I) is present in amounts of about 15% by weight, although more or less water can be used.

The term "polar organic solvent" includes solvents such as organic amides, lower alkanols, dimethyl sulfoxide, tetrahydrofuran, acetonitrile, acetone, dioxane, and the like, or a mixture of such solvents.

Organic amide solvents, such as formamide, acetamide, and dimethylformamide, are especially useful

solvents in the process of this invention. For example, the compound of formula (I) is dissolved in formamide with warming; about two volumes of water per volume of formamide are added to the formamide solution; hydrochloric acid is added until the solution has a pH of about 2; and the hydrochloride salt of formula (II) is allowed to crystallize.

Lower alkanols, such as methanol, ethanol, and isopropanol, are also especially useful solvents in the process of this invention. Ethanol is particularly advantageous because it is relatively non-toxic. When ethanol is used, typically the compound of formula (I) is slurried in warm ethanol; concentrated hydrochloric acid is added to the slurry, dissolving the compound of formula (I) in the solution which forms; and the hydrochloride salt of formula (II) is allowed to crystallize. If desired the required amount of hydrochloric acid can be added to the solvent prior to adding the compound of formula (I).

Concentrated hydrochloric acid is preferably used in the process of this invention since more dilute solutions of the acid result in larger volumes of crystallization solution. It is especially desirable to avoid larger volumes, whenever possible, in large scale crystallizations. Hydrochloric acid solutions having a concentration of about 12 N are especially useful.

The process of this invention is carried out conveniently by warming the solution containing the hydrochloride salt of formula (II) to temperatures of about 30° to about 45°C. initially and subsequently cooling it to room temperature or below.

After crystallization, the hydrochloride salt crystals can be separated by filtration, centrifugation, or other suitable separation methods. After separation, the salt should be washed to remove any remaining mother liquor. Yields of crystalline hydrochloride salt realized from this process are generally in the range of from about 80 to about 90%.

The crystalline hydrochloride salt thus obtained generally contains water in amounts from about 2% to about 15%, with the average amount of water being about 5%. Except for the presence of water, the crystalline hydrochloride salt of this invention is obtained in substantially pure form, i.e. substantially free from impurities. The term "substantially pure" as used herein means that, after correcting for the presence of water, at least 90% of the product obtained is the compound of formula (II).

The process of this invention is especially useful for purifying the compound of formula (I). Although the compound of formula (I) crystallizes, it does not crystallize as readily as the hydrochloride salt of formula (II). Because of this, it is advantageous to crystallize the hydrochloride salt of formula (II), separate it from the impurities, and then prepare the free base of formula (I) from the separated salt.

Methods suitable for separating the crystalline salt, such as filtration, are known in the art. Methods suitable for preparing the free base from the crystalline salt are also well known. The free base can be prepared by treating the hydrochloride salt with

base, but this procedure is slow because the free base is very insoluble. A preferred method of preparing the free base, therefore, comprises adding the crystalline hydrochloride salt of formula (II) which has been separated but not dried to an aqueous bicarbonate solution, maintaining the pH between about 6 and about 7, filtering to separate impurities, and precipitating the free base of formula (I) by careful addition to the filtrate of hydrochloric acid, preferably 4 N hydrochloric acid.

The following examples further illustrate the invention herein described.

EXAMPLE 1

Crystallization of the Hydrochloride Salt

10 g of the compound of formula (I) was dissolved with warming in 25-30 ml of formamide. To this solution, 50 ml of water was added. The resulting solution was acidified by the addition of 12 N HCl until the solution had a pH of about 1.5-2.0. This solution was cooled in a refrigerator for several hours until crystallization appeared complete. The crystals which formed were separated by filtration, washed with acetone, and air-dried to give the crystalline hydrochloride salt of formula (II) (80% yield).

EXAMPLE 2

On a larger scale the procedure described in Example 1 was repeated, using 1 kg of the compound of formula (I) and obtaining 810 g of crystalline hydrochloride salt of formula (II). A portion of this lot was recrystallized using the same procedure to give

highly purified crystalline hydrochloride of formula (II). This material had a purity of 99.3%, after correcting for 11.1% water content.

EXAMPLE 3

Alternate Crystallization of the Hydrochloride Salt

25 g (0.0628 m) of the compound of formula (I) was added to 100 g of ethanol at about 35°C., stirring under nitrogen. 6.29 ml (.075 m) of concentrated HCl (special grade 1.18 g/ml) was added. The resulting suspension became a thick solution when warmed to 40°C., and crystallization began to occur. The mixture was stirred slowly for 3 hours at room temperature to permit complete crystallization. The crystals which formed were separated by filtration, washed with acetone, and air-dried for 2 hours at 45°C to give 22.48 g of the crystalline hydrochloride of formula (II) (82.4% yield).

The above procedure, carried out on a larger scale, gave the crystalline hydrochloride of formula (II) in 91.7% yield. UV: A 450 = 0.165 (5% solution in methanol); $E_{1\ cm}^{1\%}$ = 341; 10.06% water (Karl Fisher).

EXAMPLE 4

Purification of the Free Base

A sample from a lot of free base of formula (I) was assayed and found to be 91.9% pure. 6.29 g (.0158 m) of free base from this lot was added to 28 ml of 2B ethanol at room temperature, under nitrogen. The mixture was warmed to 47°C. 1.58 ml (.0185 m) of concentrated HCl (1.18 g/ml) was added quickly in one portion. The HCl added amounted to about 1.2 equivalents per equivalent of free base. The temperature of the mixture rose to 50°C, and about one minute after addition of the HCl, the heterogeneous mixture turned homogeneous. About two minutes after addition of the HCl, the hydrochloride salt, began to precipitate as white crystals. The mixture was allowed to cool gradually to room temperature over a period of 30 minutes. Then, over a further 30 minute period, the mixture was cooled to below 10°C, and it was kept below 10°C for a further 30 minute period. The mixture was then filtered, and the cake of crystalline hydrochloride salt of formula (II) was washed and dried.

The dried cake of crystalline hydrochloride salt and 7 mole-% $NaHCO_3$ solution were simultaneously added, portionwise, to a flask containing 60 ml of 20 mole-% $NaHCO_3$, so that a pH of 6.5 to 7.5 was maintained. The resulting solution was filtered, and the filtrate was poured into a 250 ml flask. About 18 ml of 1N HCl was added to the solution to lower the pH to about 3. The solution was seeded with granular free base of formula (I), and crystallization began. The pH of the solution was maintained below about 4.5 by addition of 1N HCl, as required. After 1 hour at room temperature the crystals of the free base were separated by filtration, washed with about 60 ml of deionized water, and vacuum dried at 50°C. The yield was 4.63 g; 0.9% water (Karl Fisher). A sample was assayed and found to be 96.2% pure.

## CLAIMS

1. A crystalline hydrochloride salt of the formula

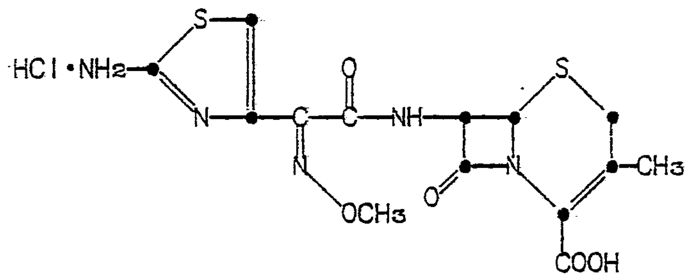

(II)

wherein the oxime is in the *syn* configuration.

2. A process for preparing a crystalline hydrochloride salt of the formula

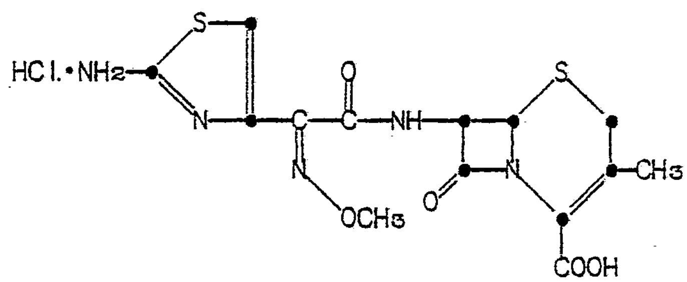

(II)

wherein the oxime is in the *syn* configuration, which comprises the steps of

(a) mixing a compound of the formula

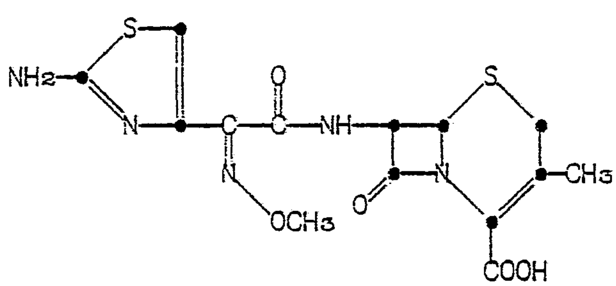

(I)

wherein the oxime is in the *syn* configuration, with hydrochloric acid in a solvent selected from water,

polar organic solvents and mixtures thereof; and

(b) separating crystals of the hydrochloride salt of formula (II) from the mixture.

3. The process of claim 2 wherein the solvent used is aqueous and the amount of hydrochloric acid used is sufficient to maintain the pH of the mixture between about 1.5 and 3.5.

4. The process of claim 3 wherein step (a) comprises dissolving the compound of formula (I) in formamide with warming, adding two volumes of water per volume of formamide to the solution, and adding hydrochloric acid to the solution until the pH is about 2.

5. The process of claim 2 wherein about 1 to 2 equivalents of hydrochloric acid is used per equivalent of compound of formula (I).

6. The process of claim 2 or 3 wherein the solvent includes formamide.

7. The process of claim 2 or 3 wherein the solvent includes ethanol.

8. The process of claim 2 wherein

in step (a) the compound of formula (I) is added to ethanol, hydrochloric acid is added to the mixture, and the mixture is stirred at a temperature between about 30° to about 45°C until the compound of formula (I) is in solution; and

in step (b) the solution is cooled to room temperature or below to promote crystallization of the compound of formula (II).

9. A crystalline hydrochloride salt of the formula

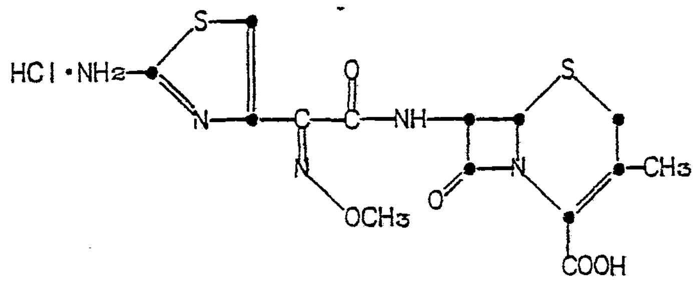 (II)

wherein the oxime is in the *syn* configuration, for use as an antibiotic.

10. A pharmaceutical formulation which comprises as an active ingredient a compound of the formula

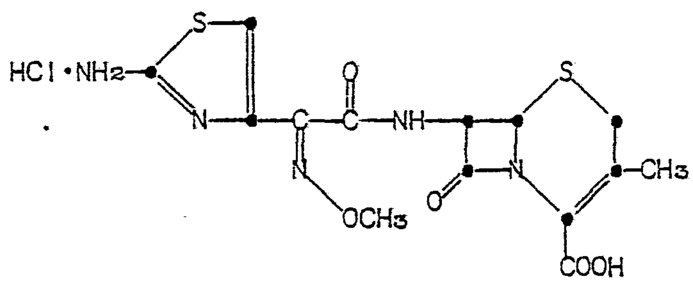 (II)

wherein the oxime is in the *syn* configuration as claimed in claim 1, associated with one or more pharmaceutically-acceptable excipients or carriers therefor.

11. A process for purifying an impure lot of compound of the formula

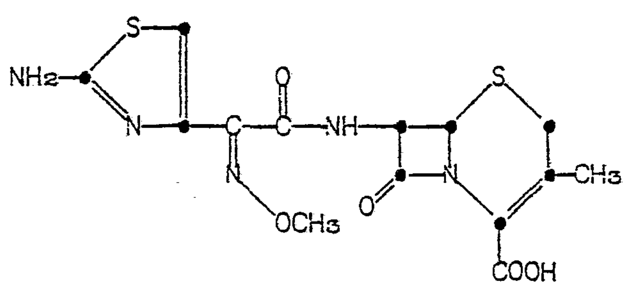 (I)

wherein the oxime is in the *syn* configuration which comprises:

(a) mixing the impure lot with hydrochloric acid in a solvent selected from water, polar organic solvents, and mixtures thereof;

(b) separating from the mixture crystals of the hydrochloride salt of the formula

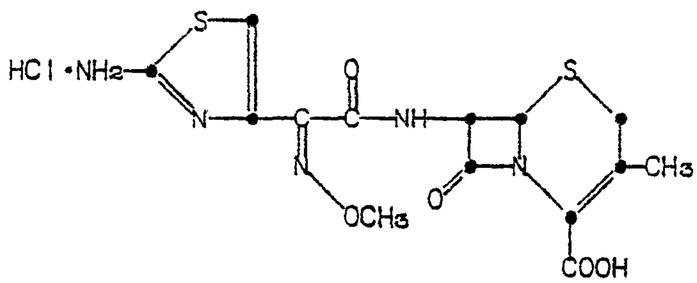

(II)

wherein the oxime is in the *syn* configuration;

(c) dissolving the crystalline hydrochloride salt of formula (II) in an aqueous bicarbonate solution with the pH maintained between about 6 and about 7;

(d) adding hydrochloric acid to the solution to precipitate the compound of formula (I); and

(e) separating the compound of formula (I) from the mixture.